# EUROPEAN PATENT SPECIFICATION

(11) **EP 4 146 294 B1**
(45) Date of publication and mention of the grant of the patent: **11.11.2026**
(21) Application number: 21727946.2
(22) Date of filing: 03.05.2021
(51) Int. Cl.: A61L 24/08, A61L 26/00, A61K 31/717, A61K 45/06, A61L 24/00, A61L 31/04, A61L 31/14, A61P 41/00

(54) **COMPOSITIONS COMPRISING OXIDIZED CELLULOSE**
ZUSAMMENSETZUNGEN MIT OXIDIERTER CELLULOSE
COMPOSITIONS COMPRENANT DE LA CELLULOSE OXYDÉE

(30) Priority: 05.05.2020 US 202016866739
(43) Date of publication of application: 15.03.2023
(73) Proprietor: Omrix Biopharmaceuticals Ltd., Rehovot 7610601 (IL)
(72) Inventor: ILAN, Erez, 7039500 Kibbutz Netzer Sereni (IL); FAINGOLD, Omri, 7634940 Rehovot (IL); MONTIA, Eve, 7632848 Rehovot (IL)
(74) Representative: Carpmaels & Ransford LLP
(86) International application number: PCT/IL2021/050513
(87) International publication number: WO 2021/224919

(56) References cited:
- WO-A1-2013/177242
- WO-A1-2017/079059
- US-A- 4 668 224
- US-A- 5 134 229
- US-B1- 6 627 749

## Description

### FIELD OF THE INVENTION

The invention relates to oxidized cellulose powders for use in adhesion prevention at a bleeding site.

### BACKGROUND OF THE INVENTION

In various situations during surgery, therapeutic agents are spread inside the human body, for prevention of bleeding or adhesions after surgery. Existing agents have drawbacks, for example, sheets are difficult to place in minimal invasive surgeries (MIS).

Adhesion prevention and control of bleeding are essential and critical in surgical procedures to minimize blood loss, to reduce post-surgical complications, and to shorten the duration of the surgery in the operating room.

The selection of appropriate methods or products for the control of bleeding and/or adhesions is dependent upon many factors, which include but are not limited to bleeding severity, anatomical location of the source and the proximity of adjacent critical structures, whether the bleeding is from a discrete source or from a broader surface area, visibility, and precise identification of the source and access to the source.

Several products are commercially available for preventing adhesions formation. Some of the adhesion barriers are based on oxidized cellulose (OC), such as oxidized regenerated cellulose (ORC), modified sugars and modified starch.

In an effort to address the above-described problems, materials have been developed for both adhesions prevention and controlling excessive bleeding. Topical Absorbable Hemostats (TAHs) are widely used in surgical applications. TAHs encompass products-based OC, gelatin, collagen, chitin, chitosan, etc. To improve the hemostatic performance, scaffolds based on the above materials can be combined with biologically-derived clotting factors, such as thrombin and fibrinogen.

Due to its biodegradability and its bactericidal and hemostatic properties, OC based materials such as ORC, have long been used as topical hemostats. Products based on ORC are used in a variety of surgical procedures, including: neurosurgery, abdominal surgery, cardiovascular surgery, thoracic surgery, head and neck surgery, pelvic surgery, gynecologic surgery, and skin and subcutaneous tissue procedures. Several methods for forming various types of hemostats based on OC materials are known, whether made in powder, woven, non-woven, knit, and other forms. Currently utilized hemostats include powder, or fabrics comprising ORC.

There is a need for improved forms and materials which facilitate ease of application, especially in bleeding sites and in hard-to-reach areas.

US9447196B2 discloses a process for dissolving modified cellulose including contacting a modified cellulose solution with at least one non-solvent to form a plurality of modified cellulose particles.

US9572907 describes implantable medical devices containing a polymeric film layer containing glycerol and at least one biopolymer.

EP3258974 describes a hemostatic composition comprising water-retaining, binder dust suppression, inorganic and organic hemostatic agents.

US6627749 discloses a controlled chemical method to produce oxidized cellulose in high yields (75-95%) and different levels of oxidation (carboxyl content <25.6%, w/w), suitable for use as an immobilizing matrix or carrier for drugs, chemicals, and biological macromolecules.

US20060008505 discloses a delivery system for a hemostatic material comprising a self-adhesive strip of a bio-adhesive, especially pectin, and a glycerol plasticizer.

WO2013049049 discloses an adhesion prevention fabric having a first absorbable oxidized regenerated cellulose nonwoven fabric and a second absorbable oxidized regenerated cellulose woven or knitted fabric; and an adhesion prevention fabric that is useful for adhesion prevention in the presence of blood.

US5134229 relates to an oxidized cellulose material neutralized by contacting an acidic oxidized cellulose material with a water and alcohol solution of a basic salt of a weak organic acid to elevate the pH of the cellulosic material to between 5 and 8. The resulting neutralized product is said to be storage stable and have therapeutic applications, including hemostasis and adhesion prevention.

INTERCEED (Johnson & Johnson Patient Care Inc., New Brunswick, NJ) is an absorbable fabric specially designed to reduce postsurgical adhesions (FERTILITY AND STERILITY Vol. 51, No.6, June 1989 INTERCEED (TC7) Adhesion Barrier Study Group).

### SUMMARY OF THE INVENTION

The present invention relates to compositions comprising oxidized cellulose (OC), e.g., oxidized regenerated cellulose (ORC), in the form of a powder for use as an adhesion prevention material in the presence of blood, wherein the carboxyl content of the OC ranges from 9% to 18%, by weight..

The present invention is based, *inter alia,* on the unexpected finding that oxidized OC, e.g., ORC in the form of a powder can be effective for adhesion prevention even in the presence of blood. The carboxyl content of the OC may be equal to or below 18% by weight. This finding is surprising as it was previously reported that the effectiveness of INTERCEED in adhesion prevention is limited in the presence of bleeding.

The carboxyl content of the OC is equal to or below 18%, by weight. The composition is for use as an adhesion prevention material in a bleeding site. In some embodiments, the powder is in an aggregated form (i.e. in the form of one or more aggregates). In some embodiments, the powder comprises milled OC or ORC.

In some embodiments, the powder is in an aggregated form, the OC comprises ORC, and the carboxyl content of the OC or ORC is equal to or below 18%, by weight.

In some embodiments, the particle size of at least 90% of the powder ranges from 10 to 2,000 µm, optionally from 50 to 300 µm.

In some embodiments, the composition is characterized by adhesion prevention potency of at least 120% as compared to OC fabric having or consisting of same carboxyl content. In some embodiments, the powder is gamma irradiated.

In some embodiments, the OC comprises ORC in the form of compacted powder, optionally comprising particles having average aspect ratio from about 1 to about 18.

In some embodiments, the powder has average particle size of 1.75 microns to 116 microns with a median size of about 36 microns. In some embodiments, the compacted ORC powder is roller compaction processed ORC powder, and/or hammer mill processed ORC powder. In some embodiments, the aggregates of ORC (e.g., made by milling) are compacted.

In some embodiments, the composition further comprises an additive, wherein the additive may comprise carboxymethyl cellulose (CMC) or other polysaccharides, calcium salt, anti-infective agent, hemostasis promoting agent, gelatin, collagen, or combinations thereof.

In some embodiments, the composition is in the form of particulate aggregates, wherein the OC forms interconnected individual cellulosic fibrils having in aggregate form a sphericity of at least 0.6, a dimension along its longest axis that is less than about 500 microns and greater than about 50 microns. In some embodiments, the aggregates have a size distribution profile with D15 greater than about 80 microns, D50 from about 140 to 250 microns, D90 less than about 370 microns, a bulk density greater than 0.45 g/mL, and/or sphericity (sh50) equal or greater than 0.7.

The composition may be provided in a kit comprising: a. a container containing the composition ; b. an applicator for applying the composition to a tissue; and c. optionally instructions for use. In some embodiments, the container is comprised in the applicator.

In some embodiments, the OC comprises ORC.

By "preventing tissue adhesion" it is meant to encompass prevention of tissue adhesion within the tissue (on which the composition has been applied), or preventing tissue adhesion of adjacent tissues, wherein the blood is present in one or in both of these tissues.

In some embodiments, the carboxyl content of the ORC is equal to or below 18%.

In some embodiments, the composition further comprises at least one biologically active agent. In some embodiments, the at least one biologically active agent is or comprises calcium. In some embodiments, the composition further comprises one or more excipients selected from the group consisting of sodium chloride, mannitol, albumin, and sodium acetate.

In some embodiments, the OC powder comprises ORC powder.

Typically, powder is matter in a finely divided state, such as particulate matter. Powder can be a loose grouping or aggregation of solid particles, typically, but not exclusively, smaller than 1000 micrometers.

Absorbable oxidized regenerated cellulose fabrics that may be used to prepare the powder is knitted and includes but is not limited to INTERCEED, available from Johnson & Johnson Wound Management, a division of Ethicon, Inc., Somerville, NJ.

Absorbable oxidized regenerated cellulose nonwoven, woven or knitted fabrics may be used to prepare the powder. Such fabrics, for example, are described in U.S. Pat. Nos. 4,626,253, 5,002,551 and 5,007,916.

In some embodiments, in order to achieve adhesion prevention, the carboxyl content of the OC is equal to or below 18% e.g., as per United States Pharmacopeia (USP) 23-NF18.

Hereinthroughout, the carboxyl content of the OC is by weight %.

Prevention may be achieved by administration of a compound to a subject prone to develop adhesions.

In some embodiments, the carboxyl content of the OC (or ORC) is equal to or above 9% and equal to or below 18% as per United States Pharmacopeia (USP) 23-NF18.

In some embodiments, the carboxyl content of the OC (or ORC) is equal to or above 9% and equal to or below 18% as per United States Pharmacopeia (USP) 23-NF18.

In some embodiments, the carboxyl content of the OC (or ORC) is equal to or above 12% and equal to or below 18% e.g., as per United States Pharmacopeia (USP) 23-NF18.

Suitable ORC fabrics include absorbable adhesion barriers such as, without being limited thereto, INTERCEED absorbable adhesion barrier available from Ethicon, Inc., Somerville, N.J.

In some embodiments, the fabric is a warp knitted tricot fabric constructed of bright rayon yarn that is subsequently oxidized to include carboxyl or aldehyde moieties in amounts effective to provide the fabrics with biodegradability. The fabric may be oxidized by reacting the cellulose with a solution of nitrogen dioxide in a perfluorocarbon solvent as described by F. Boardman et al. in US Patent Number 5,180,398. In another embodiment, the carboxyl content ranges from about 12% to about 18% (weight/weight). In yet another embodiment, the oxidized regenerated cellulose degree of oxidation ranges from about 9.5% to about 10.5% (weight/weight). In some embodiments, the degree of oxidation is about 9% to about 18% (weight/weight) as per United States Pharmacopeia (USP) 23-NF18.

In some embodiments, the carboxyl content of the OC powder is equal to or above 9% as per United States Pharmacopeia (USP) 23-NF18. In some embodiments, the carboxyl content of the OC powder is equal to or above 9% and equal to or below 18% (weight/weight) as per United States Pharmacopeia (USP) 23-NF18. In some embodiments, the carboxyl content of the OC powder is equal to or above 12%. In some embodiments, the carboxyl content of the OC powder is equal to or above 12% and equal to or below 18% (weight/weight) as per United States Pharmacopeia (USP) 23-NF18. In another embodiment, the degree of oxidation ranges from about 12% to about 18% (weight/weight).

In yet another embodiment, the oxidized regenerated cellulose woven fabric has a degree of oxidation ranging from about 9.5% to about 10.5%..

The composition is for use as an adhesion prevention material in the presence of blood.

In some embodiments, the compositions in which the carboxyl content of the OC is about 12% to about 18% are for use for preventing adhesion in bleeding sites e.g., in soft tissues. Adhesion prevention may be achieved by administration of the composition in an embodiment thereof in or adjacent to a bleeding tissue e.g., in a wound.

In some embodiments, the particle size of the OC powder is between 10 µm and 2,000 µm, optionally between 50 µm and 300 µm. In some embodiments, the carboxyl content of the OC in the powder is about 12% to about 18%. Further disclosed is that the composition is not flowable at one or more temperature values selected from the group consisting of 10°C, 15 °C, 20°C, 25 °C, 30 °C, 35°C, 37°C, and 40°C.

In some embodiments, the total water content in the composition is less than about 8% w/w.

In some embodiments, the composition disclosed hereinthroughout is an adhesion prevention powder for use in a bleeding site, comprising OC having a carboxyl content of equal to below 18% (by weight) (e.g., 9%, 10%, 11%, 12%, 13%, 14%, 15%, 16%, 17%, or 18%, including any value and range therebetween), characterized by adhesion prevention potency of at least 120%, or at least 150% as compared to OC fabric having or consisting of a similar carboxyl content. Herein, by similar it is meant the same or ± 10%. In some embodiments, the powder is radiated powder, e.g., gamma radiated powder.

In one embodiment, the adhesion prevention powder for use in a bleeding site comprises milled OC (e.g., ORC) in an aggregated form. In some embodiments, the carboxyl content of the OC powder in milled OC having aggregated form for use in a bleeding site is equal to or above 9% and equal to or below 18% (by weight) as per United States Pharmacopeia (USP) 23-NF18. In some embodiments, the carboxyl content of the OC powder in milled OC having aggregated form for use in a bleeding site is equal to or above 9% and equal to or below 18% (by weight) as per United States Pharmacopeia (USP) 23-NF18. In some embodiments, the carboxyl content of the OC powder in milled OC having aggregated form for use in a bleeding site is equal to or above 12% and equal to or below 18% (by weight) as per United States Pharmacopeia (USP) 23-NF18.

The term "bleeding site" also encompasses a situation in which the bleeding does not necessarily originate from the site but rather from other site or origin, such as tissue that may be e.g., adjacent thereto. Accordingly, the terms "in a bleeding site" and "in the presence of blood" are used hereinthroughout interchangeably.

Absorbable oxidized regenerated cellulose non-woven, woven or knitted fabrics may be used to prepare the powder.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawing. With specific reference now to the drawing in detail, it is stressed that the particulars shown are by way of example and for purposes of illustrative discussion of embodiments of the invention. In this regard, the description taken with the drawing makes apparent to those skilled in the art how embodiments of the invention may be practiced.

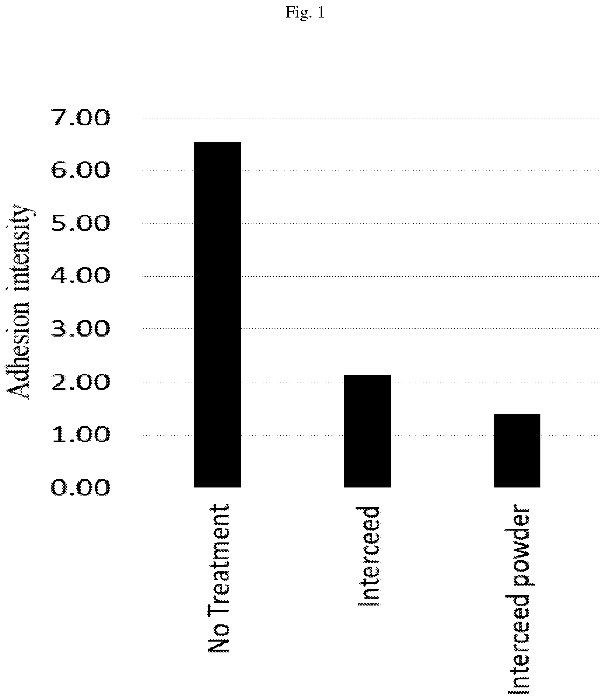

Fig. 1 presents graphs showing adhesion intensity in a rat cecal abrasion model treated with compositions comprising INTERCEED^{®} ORC and glycerol. Left to right: No treatment, INTERCEED^{®}, INTERCEED^{®} powder (milled/ground ORC which was compressed into small granules), n=6.

### DESCRIPTION OF EMBODIMENTS OF THE INVENTION

An object of the present invention is to provide a composition comprising oxidized cellulose (OC), for use as an adhesion prevention material, which may easily be applied to a bleeding site of need, especially in difficult to reach areas of the body. A further advantage of the compositions of the invention is that they are bioabsorbable, and therefore may be left behind following surgery without causing any side effects.

The present invention is therefore based on the surprising finding that OC powder at a certain range of oxidation degree may be applied to a site of need in order to obtain biological activity, such as adhesion prevention.

By "applied to a site of need" it is meant to refer e.g., to a topical application of the composition at the site, e.g., at a bleeding surgical site of a tissue, or adjacent thereto, to prevent adhesions.

Reference is made to **Fig. 1****,** which shows a comparison of adhesion prevention using 1- INTERCEED^{®} fabric, 2- milled/ground INTERCEED which was compressed into small granules (INTERCEED powder), tested in a rat cecal abrasion model. It was found that INTERCEED powder exhibits adhesion prevention activity better than or comparable to INTERCEED sheet at a bleeding site. It is further noteworthy that gamma radiated INTERCEED aggregated powder exhibited adhesion prevention potency of about 135% as compared to INTERCEED fabric.

INTERCEED powder in the form of aggregates is also named herein: milled /ground INTERCEED in aggregated form, milled/ground INTERCEED which was compressed into small granules, compacted INTERCEED powder, and INTERCEED compacted in the form of aggregate.

INTERCEED powder in the form of aggregates may be generated by the process described in US Patent 9,539,358 Examples. Oxidized Regenerated Cellulose (ORC) is the base material in INTERCEED^{®} sheets (Ethicon) which may be used instead of SURGICEL^{®} sheets (Ethicon). INTERCEED powder in the form of aggregates may be subjected to 20-45 kilogray of gamma radiation (e.g., by Sorvan Radiation LTD technique) to provide sterility.

As used herein, and unless stated otherwise, the terms "by weight", "w/w", "weight percent", or "wt. %", which may be used herein interchangeably describe the concentration of a particular substance out of the total weight of the corresponding mixture, solution, formulation, or composition.

As used herein, the term "bleeding" refers to extravasation of blood from any component of the circulatory system. A "bleeding" thus encompasses unwanted, uncontrolled and often excessive bleeding in connection with surgery, trauma, or other forms of tissue damage, as well as unwanted bleedings in patients having bleeding disorders.

As used herein, the term "adhesion" or "tissue adhesion" refers to connection of tissues not normally connected. For example, adhesions can occur as post-operative complication.

As used herein, the terms "controlling", "preventing", or "reducing", which may be used herein interchangeably in the context of the tissue adhesion, including any grammatical inflection thereof, indicate that the formation of tissue adhesion is completely or partially prevented, or the severity of the adhesion is lower, for example according to the adhesion evaluation scheme according to Poehnert et al., 2015, International Journal of Medical Sciences 12(1):1-6, described in the examples section.

By "around room temperature" it is meant to refer to at least one temperature value within the range of 10°C to 40°C, or 15°C to 37°C. e.g., 10°C, 15°C, 20°C, 25°C, 30°C, 35°C, 37°C, or 40°C, including any value therebetween.

Non-limiting exemplary powders comprise solid particles comprising ORC fiber and/or granules (also termed "granulated ORC").

As used herein, by "homogeneous" it is meant to refer to a uniform composition and texture throughout.

The term "oxidized cellulose" (or "OC") refers to a cellulose derivative in which at least some of the primary alcohol groups, e.g., on the carbon 6 of the anhydroglucose unit is oxidized to a carboxylic acid, and is optionally functionalized.

OC may be produced by applying an oxidizing agent on cellulose. The oxidizing agent may be selected from, without being limited thereto, chlorine, hydrogen peroxide, peracetic acid, chlorine dioxide, nitrogen dioxide, persulfates, permanganate, dichromate-sulfuric acid, hypochlorous acid, hypohalites, periodates, or any combination thereof, and/or a variety of metal catalysts. Oxidized cellulose may contain carboxylic acid, aldehyde, and/or ketone groups, instead of, or in addition to the original hydroxyl groups of the starting material, cellulose, depending on the nature of the oxidant and reaction conditions.

The OC used in the compositions for use of the invention is in the form of a powder (also referred to as granulated OC, milled/ground OC, or milled/ground OC in the aggregated form). The milled/ground OC may be prepared by various methods including from existing products, and some non-limiting examples of such products are described below. As some of the existing products are in the form of a fabric, the OC powder may be prepared by grinding or milling the fabric to obtain a powder. For example, milled OC (or ORC) may be obtained by reducing the size of an OC sheet, such as a SURGICEL^{®} or an INTERCEED^{®} sheet, by milling, as described in US Patent 9,539,358 disclosing a preparation of compacted Powders comprising ORC-Ball-Milled powders (BMP).

Accordingly, in some non-limiting exemplary embodiments, several pieces of 4"×4" pre-trimmed non-sterile INTERCEED fabric (ETHICON, Inc., Lot #7A8654) can be vacuumed dried for 24 hours prior to milling. Six-gram samples can be mixed with 12 high-density ZrO₂ balls (20 mm in diameter; Glen Mills Inc., Clifton, N.J., USA) and sealed in a 250 mL grinding jar. The jar can be clamped into the latching brackets and then counterbalanced on the mill (planetary ball mill PM100; Retsch, Inc., Newtown, Pa., USA). Milling can be carried out at 300 rpm for 10 min. The milled powder then can be dried in a vacuum oven (Fisher Scientific Model 280A Isotemp vacuum oven) with a vacuum pump (LabCare America Pump PV-35) at 65° C. for 2.5 h. The milled powder may be finally stored in a nitrogen box. Similar methods as above can be used to prepare powder with ORC-based SURGICEL^{®}NU-KNIT^{®} absorbable hemostat. Roller-Compacted ORC powder can be prepared by using ORC shredded through a Fitz Mill equipped with a screen mesh 1726-150. The shredded ORC powders can be fed into a roller compactor (WP 120x 40V, #900-0071, Alexanderwerk, Inc, PA) and be compacted as described in US patent 9,539,358.

Additionally or alternatively, the powder compositions for use according to the invention comprising the fibers and optionally other compounds are compacted in the form of aggregates, optionally using steps of drying, milling/grounding and sieving e.g., as described in US patent 10,034,957. The sieve used defines the particle size of the powder.

Accordingly, as described in US patent 10,034,957, the milling step may be preceded by a step of slitting and cutting the cellulosic source material forming pieces. The milling step may be a two-part process with the second part being performed in an air classifier wherein the second part can be repeated three times. The intermediate fine fiber produced by this process may have a size distribution with D50 of less than about 100 microns and D90 of less than about 180 microns. The intermediate fine fibers may be humidified to water content of between 11.0% and 20% by weight. The intermediate fine fibers may be roller compacted material and then may be subjected to pre-breaking and subsequently followed by a step of final milling. The intermediate fine fibers may be compacted at a roller pressure of at least 130 bars. The intermediate fine fibers may be compacted at a roller force of at least 26.0 kN/cm. The resulting materials may be aggregates fraction having dimensions along their longest axis of 75-300 µm by screen sieving method. In some embodiments, the aggregates fraction is characterized by a size distribution such that D15 is greater than about 80 microns, D50 is from about 140 to 250 microns and D90 is less than about 370 microns. As described hereinthroughout, the source materials may be selected from ORC fabric, ORC non-woven fabric, shredded ORC material or combinations thereof. The source materials may further comprise an additive or an active agent selected from, without limitation, carboxymethyl cellulose, a calcium salt, an anti-infective agent, a gelling agent, gelatin, collagen, or combinations thereof.

In some embodiments, one process for manufacturing the inventive hemostatic aggregates comprises the steps of: a) slitting and cutting of cellulosic source material; b) milling the resulting material from step a); c) a second milling step in an air classifier; d) humidification; e) roller compaction; f) sieving; g) dehumidification or drying; h) optional dosing into storage containers or into delivery devices, primary packaging and secondary packaging; and i) optional sterilization.

Slitting and cutting may be performed to slit and cut fabric into appropriate size pieces that are between approximately 1 inch by 3 inches or 2 inches by 3 inches, though smaller pieces may also be used. The main operations performed for slitting and cutting are to unwind a roll of fabric, slit the fabric into strips, cut the strips to size and deliver the cut pieces into the first milling step. A number of cutting and slitting machines are known and commercially available, such as, without limitation, AZCO Model FTW-1000 available from AZCO.

In some embodiments, in the first milling step, processed pieces of cellulosic fabric are converted from an intermediate coarse fiber produced in the slitting and cutting step to a material having D90 value of less than 452 µm and D50 value of less than 218 µm, while having minimal impact on the color index and water-soluble content of the material. A number of machines for milling are commercially available, such as, without limitation, Models DASO6 and WJ-RS-D6A manufactured by Fitzpatrick, which are hammer mill type milling machines, equipped with a 497 micron round screen and a set of blades that breaks down the fabric until it passes through the screen to produce intermediate coarse cellulosic fiber. In an exemplary processing run, mill speed may be about 7000 RPM; processing temperature at less than 80 °C; screen size between 1534 and 9004; number of blades as 8 (2 impellers each); blade type as a 225 knife, impact type blades; blade orientation set as "impact". Intermediate coarse fiber from the first milling step may be fed at a controlled rate into the second mill and pass through two milling chambers that are separated by a milling screen. The material may be pulled through the milling chamber by an air blower. The intermediate coarse fiber may be processed through the air classifier equipment three times in order to obtain the desired size. At the end of the second milling step, the intermediate fine fiber may be collected.

In an exemplary processing run, a Quadro Air Classifier F10 may be used in the second milling step with a milling speed of 8400 rpm, blower speed of 1800 rpm, 0.0018" round hole screen and 3 passes. ORC intermediate fine fiber may be also produced in one step by ball milling instead of the two steps milling steps as described above. In an alternative ball milling embodiment, 50 g of pre-cut ORC fabric (2"×2") is ball milled with 12 high-density Zirconia (zirconium dioxide ZrO₂, 20 mm in diameter; Glen Mills Inc., Clifton, N.J., USA) by placing the balls and the samples in a 500 mL grinding jar. The jar may be clamped into the latching brackets and then counterbalanced on the planetary ball mill PM100; Retsch, Inc., Newtown, Pa., USA). The milling may then be performed bi-directionally at 450 rpm for 20 minutes.

In some embodiments, following the milling process, the resulting cellulosic intermediate fine fiber is humidified to moisture content between of about 11% and about 18%, or between 11% and about 16%, or about 12% to 16% for the subsequent processing, including a roller compaction process. A non-limiting humidity chamber suitable for the humidification step is commercially available as Model CEO-916-4-B-WF4-QS by Thermal Product Solutions. Humidification of chamber air may be achieved by water vapor injection. The typical steady-state temperature of 25 °C may be utilized, while the humidity level may be cycled between 75% and 85%, with a target of e.g., 85% air humidity. Humidification time or residence time of the material inside the humidity chamber may range from several hours to several days depending on the quantity of the material and air recirculation. In a typical cycle, the material may have 12-13 hours residence time for about 3,000 grams of cellulosic intermediate fine fiber arranged in several trays and exposed to 85% relative humidity and a target of 12% moisture content of the powder after humidification; use of cellulosic intermediate fine fiber with a moisture content fed into the compaction step that is greater than 16%, such as a moisture content of 20% by weight; the resulting ORC intermediate fine fiber may be caked during compaction, exhibits very poor flowability, and jams the compactor. Thus, high humidity of the intermediate fine fiber may not result in suitable hemostatic aggregate materials. Conversely, when the moisture content of the intermediate fine cellulosic fiber is lower than about 8%, the yield of hemostatic aggregates is extremely low, somewhere about 5% yield of desired hemostatic aggregates. Humidified intermediate fine ORC fiber may then be compacted and sieved to obtain hemostatic aggregate materials. The roller compactor may compact the feed, which may then be subjected to pre-breaking, final milling, and sieving in a screener to obtain the desired hemostatic aggregates sizes.

Compaction equipment is known and commercially available. Non-limiting exemplary compaction units are the Fitzpatrick Chilsonator IRR220-L1A with Retsch manual sieving AS200 Screener and the Fitzpatrick Chilsonator CCS220/M3B & RV-M5A with Screener Sweco Vibro-energy unit integrated under M5A. The compaction processing may be performed using two separate subsystems that are bound by a common electrical system. For example, a first subsystem (Roller Compactor: main unit) may be the Fitzpatrick Chilsonator CCS220 roller compactor and the M3B mill for pre-breaking the compacted material, while the second subsystem (Roller Compactor: secondary milling unit) may be M5A mill for the final milling with a Sweco or Retch screener for the separation to obtain the desired size aggregates. Humidified intermediate fine cellulosic fiber may be fed into the hopper of the roller compactor unit, first passed through a main milling unit and then proceed on through a second milling unit. A container may be provided to capture the pre-broken cellulosic material resulting from the main milling unit. The pre-broken pieces of cellulosic material may then be fed into the secondary milling unit, which performs the final milling and screening utilizing a screen mesh. The resulting milled cellulosic material may be separated into fines (<75 µm), targets (75-300 µm), and overs (>300µm) using a screen mesh, such as the Sweco or Retch screener described herein.

Other methods of generating compacted ORC powders of high density and low aspect ratio besides ball milling may be utilized. Rolling compaction refers to the continuous compaction of powders by roll mills. The powder may be delivered by feed screw to rolls and densified by the pressure and shear force. Roll compaction is a powder agglomeration process used in a variety of industries including the pharmaceutical, mineral and chemical industries. Roll compaction of poor flowability powder mixtures may require screw feed of the powder between two counter-rotating rolls. These may then draw the powder into the compaction zone and apply a high pressure forming a strip of compacted powder. Powders compacted to such strips or ribbons by pressure between two-counter rotating rolls may then be further milled into granules of low aspect ratio. Woven or non-woven ORC material, or shredded or ball milled ORC material may be further roller compacted to reach desired low aspect ratios and high-density ORC particles.

A hammer mill is another method that may be used to make an ORC particle having sufficient low aspect ratio and high tapped density. A hammer mill operates by impact action and will pulverize most dry, free-flowing materials. Material may be fed into the hammer mill from the top and may then fall into the grinding chamber. The material may be contacted by a series of hardened steel hammers rotating at high speed. The material may be ground by repeated contact with these hammers, contact with the walls of the grinding chamber, and particle to particle contact. The material may remain in the hammer mill grinding chamber until the particles become small enough to escape by passing through the perforated screen that covers the bottom half of the grinding chamber.

A hammer mill may be essentially a steel drum containing a vertical or horizontal rotating shaft or drum on which hammers are mounted. The hammers may be free to swing on the ends of the cross, or fixed to the central rotor. The rotor may be spun at a high speed inside the drum while material is fed into a feed hopper. The material may be impacted by the hammer bars and may be thereby shredded and expelled through screens in the drum of a selected size. The hammer mill may be used as a primary, secondary, or tertiary crusher, i.e. ORC may be hammer milled from woven or non-woven material ORC source, or from shredded or ball milled ORC material. The main difference between cutting (shredding) and ball milling and other compacting/milling processes is the crushing mechanical impaction without sharp blades which is utilized in the ball milling and other compaction processes. Due to absence of sharp blades, the particles acquire different properties in terms of particle shape, surface, tapped density, etc. which are distinct from shredded (i.e. milled utilizing blades) particles.

In exemplary embodiments, OC has been oxidized to contain carboxyl moieties in amounts effective to provide biodegradability.

U.S. Patent No. 3,364,200 discloses the preparation of carboxylic-oxidized cellulose with an oxidizing agent such as dinitrogen tetroxide in a Freon medium. U.S. Patent No. 5,180,398 discloses the preparation of carboxylic-oxidized cellulose with an oxidizing agent such as nitrogen dioxide in a per-fluorocarbon solvent. After oxidation by either method, the fabric may be thoroughly washed with a solvent such as carbon tetrachloride, followed by an aqueous solution of 50% isopropyl alcohol (IPA), and finally with 99% IPA. Prior to oxidation, the fabric may be constructed in the desired woven or nonwoven construct.

Typically, hemostats that are compatible with acid-sensitive species comprise fabric substrates prepared from a biocompatible, aldehyde-oxidized polysaccharide. In such exemplary hemostats, the polysaccharide contains an amount of aldehyde moieties effective to render the modified polysaccharide biodegradable, meaning that the polysaccharide is degradable by the body into components that are either resorbable by the body, or that can be passed readily by the body. More particularly, the biodegraded components do not elicit permanent chronic foreign body reaction when they are absorbed by the body, such that substantially no permanent trace or residual of the component is eventually retained at the implantation site.

In certain embodiments of the present invention, the OC comprises particles prepared from biocompatible, biodegradable, aldehyde-oxidized regenerated cellulose. In some embodiments, the aldehyde-oxidized regenerated cellulose is one comprising repeating units of Structure II in U.S. Pat. No. 8,709,463. In some embodiments, ORC is used to prepare an adhesion prevention material especially at a bleeding site of a tissue. Typically, regenerated cellulose is preferred due to its higher degree of uniformity versus cellulose that has not been regenerated. Regenerated cellulose and a detailed description of how to make regenerated oxidized cellulose is set forth e.g., in U.S. Patent No. 3,364,200 and U.S. Patent No. 5,180,398.

Accordingly, in some embodiments, the OC comprises ORC. Examples for OC-based products that are either in aggregated form or may be ground or milled and therefore may be utilized to prepare particles of the composition include, but are not limited to, INTERCEED^{®} absorbable adhesion barrier, SURGICEL^{®} Original absorbable hemostat, SURGICEL^{®} NU-KNIT^{®} absorbable hemostat, SURGICEL^{®} FIBRILLAR^{™} absorbable hemostat, SURGICEL^{®} SNoW^{™} absorbable hemostat and SURGICEL^{®} Powder absorbable hemostat, GelitaCel^{®} resorbable cellulose surgical dressing from Gelita Medical BV, Amsterdam, The Netherlands.

In some embodiments, the OC is derived from a plant source. It is appreciated that while the usual source for OC is plant material, OC may also be derived from a bacterial source.

In some embodiments, the cellulose for use with the present invention does not include carboxymethyl cellulose (CMC).

The compositions may be non-aqueous compositions, which means that the main liquid in the compositions is not water and the compositions have very low water content or no water at all.

In some embodiments, the water content of the composition is lower than about 8% w/w. In some embodiments, the water content of the composition is lower than about 7% w/w. In some embodiments, the total water content of the composition is lower than about 6%, 5%, 4%, 3%, 2%, 1%, 0.5%, 0.1%, or 0.01% w/w. In some embodiments, the composition is substantially devoid of water. In some embodiments, the composition does not contain water.

In some embodiments, the composition does not further comprise a solvent. In some embodiments, the composition does not further comprise an organic solvent, such as, for example, ethanol.

The composition comprises OC in the form of a powder (e.g., milled OC). As indicated above, various cellulose-based materials may be ground or milled to obtain a powder which may be used to prepare the composition.

The cellulose-based material, e.g., cellulose-based fabric, can be milled to obtain fibers that have a size distribution of D90 of less than 350 µm and of D50 of less than 167 µm as described above. If desired, the milling step may be repeated to obtain a size distribution of D90 of less than 177 µm, and D50 of less than 95 µm.

As described herein, in one embodiment, the fibers for making the composition are prepared by milling a cellulosic source material; the milling step may be preceded by forming material pieces by slitting and cutting the cellulosic source material. In this embodiment the milling step may be a two-part process with the second part performed in an air classifier wherein the second part may be repeated three times. Accordingly, after a first pass (time) in the air classifier, the resulting "long fibers" may have a size distribution of D90 of less than 350 µm and D50 of less than 167 µm. After 3 passes (3 times) in the air classifier the resulting fine ORC fibers may have a size distribution of D90 of less than 177 µm and D50 of less than 95 µm. Thus, in one embodiment, "long fibers" have a size distribution of D90 of less than 350 µm and D50 of less than 167 µm

In one embodiment of the invention, the "fine" or "short" cellulose-based fibers in the composition have a size distribution of D90 of less than 177 µm, and D50 of less than 95 µm. The cellulose-based material may be mixed or supplemented with other compounds before, during and/or after the milling steps.

In some embodiments, the disclosed aggregates are composed of a plurality of interconnected individual cellulosic fibrils that in aggregate form have a sphericity of at least 0.5, a diameter along its longest axis that is less than about 500 microns and greater than about 50 microns. The aggregates may alternatively be expressed as having a size distribution profile with D15 greater than about 80 microns, D50 from about 140 to 250 microns, D90 less than about 370 microns, a bulk density greater than 0.45 g/mL, and/or sphericity (sh50) equal or greater than 0.70. The aggregate may be characterized by having substantially no size distribution changes or minimal size distribution changes when subjected to a vibratory challenge, e.g., the size distribution profile of the hemostatic aggregates as measured by D50 does not fall below 100 microns. In one embodiment, the size distribution changes are characterized by a QICPIC optical sensor at 0.2 bars. In a still further embodiment, the size distribution changes, or minimal size distribution changes, are based to processing at 1.0 bar vacuum. In some embodiments, the aggregates have been milled, humidified, roller compacted, and dried cellulosic material.

The terms "D15", "D50", D70" and "D90" refers to 15%, 50%, 70%, and 90%, respectively (by numbers or volume), of the particles having a size that is less than or equal to the corresponding value.

As provided herein, in some embodiments, the composition is prepared from OC in the form of aggregates. The term "aggregate" describes a particle formed from assembled components.

As is further provided herein, aggregates may be optionally made by one of the following steps of humidifying the powder composition; compacting, e.g., by roller and/or slugging the powder to form aggregates; dehumidifying; milling; sieving the aggregates; and optionally dosing the resulting aggregates into a storage container or into a delivery device.

In some embodiments, the particle size of the OC is between 10 µm and 2,000 µm. In some embodiments, the particle size of the OC is between 50 µm and 1,500 µm, between 100 µm and 1,000 µm, between 100 µm and 500 µm, between 100 µm and 300 µm, between 50 µm and 1,000 µm, between 50 µm and 500 µm, or between 50 µm and 300 µm.

As provided hereinabove, in some embodiments, the OC (e.g., ORC) powder is compacted. Compacted ORC powder may be roller compaction processed ORC powder or hammer mill processed ORC powder. The disclosed powder may have a tapped density of at least 0.45 g/cm³, and/or a flowability of at least 7.5 cm/s; and/or an average particle size of 1.75 microns to 116 microns with a median size of 36 microns. In one embodiment, the disclosed powder comprises particles having average aspect ratio from about 1 to about 5; a tapped density of at least 0.67 g/cm³ and flowability of at least 70 cm/s.

In one embodiment, particulates which are compacted into high tapped density powders, have tapped density ranging from about 0.35 to about 1 g/cm³, or 0.4 to 0.9 g/cm³, such as 0.42 to 0.78 g/cm³. Particles of ORC having the specific aspect ratios may be directly made from ORC materials, such as ORC fabric or non-woven as characterized above utilizing the ball milling process. As described herein, the particle size may have overall size (largest dimension) of less than 500 microns, such as less than 300, 200, and less than 100 microns.

In another embodiment, the disclosed composition is in the form of a paste that comprises the hemostatic materials of described above and a saline solution. The paste may have a viscosity greater than 10000 Pa-s at room temperature.

In another embodiment a compacted ORC-based material in the form of a powder, having an aspect ratio of from about 1 to about 18 may be used. The ORC-based material may be a shredded ORC material. In one embodiment, the compacting is performed by ball milling as described above. In another embodiment, the compacting is performed by roller compaction or by hammer milling as described above.

The present invention is directed to the disclosed composition for use in a method of preventing adhesion at a bleeding site by applying the disclosed composition in the powder form as described above onto and/or into the bleeding site in a tissue of a subject.

Due to high tapped density and low aspect ratio of powders, the resulting ORC powders may be utilized as a material for adhesion prevention at a bleeding site in powder form with superior hemostatic properties and good tissue conformability and flowability. In addition, the ORC materials may be physically incorporated with other agents and biopolymers to improve anti-adhesion properties at bleeding sites.

The low aspect ratio (1-20) particles may comprise the majority of the particles constituting the powdered material, i.e. over 50%, such as over 80% or over 90% of particles. The particles having overall size (largest dimension) less than 500 microns, such as less than 300, 200, and less than 100 microns may comprise the majority of the particles constituting the powdered material, i.e. over 50%, such as over 80% or over 90% of particles.

The product resulting from the ball-milling process comprising low aspect ratio and high tapped density particles of ORC, as described above, are shown to have effective adhesion prevention properties.

For the purposes of the present disclosure, the aspect ratio of powder is defined as average aspect ratio of particles comprising the powder, with the aspect ratio of particles being determined by a measurement of the longest dimension of the particle (length) divided by the shortest dimension of the particle (width) as visible under appropriate magnification under electronic or optical microscope. The lowest aspect ratio (AR) of 1 corresponds to a round particle, having longest dimension equal to the shortest dimension. An aspect ratio of about 20 corresponds to a fibrous particle having length 20 times diameter. Exemplary aspect ratios according to the present invention are from 1 to 20, more specifically from about 1.5 to about 17.5.

In some embodiments, the disclosed composition having an OC with a carboxyl content of 12% to 18% is for use as an adhesion prevention composition for preventing formation of adhesions at a bleeding site of tissues. In some embodiments, the flowable or the non-flowable compositions in which the carboxyl content of the OC is 12% to 18% are for use as an adhesion prevention material, for reducing or preventing adhesions at a bleeding site of tissues.

In some embodiments, the disclosed composition in which the carboxyl content of the OC is 12% to 18% is flowable at one or more temperature value selected from the group consisting of 10°C, 15°C, 20°C, 25 °C, 30°C, 35 °C, 37 °C, and 40 °C.

The flowability of particles is a parameter that influences the deployment of powders during surgical procedures. High flowability may be preferred in a surgical setting for ease of deployment.

Bulk density is the ratio of the mass of an untapped powder sample and its volume, including the contribution of the interparticulate void volume. Tapped density is a measure of increased bulk density of powder that is obtained by mechanically tapping a container of the powder. Tapped density appears to be correlated with flowability. High tapped density may be preferred for ease of deployment and mixing. Tapped density, for purposes of this application may be measured using a modified USP 616 method in which one (1) gram of powder is introduced into a dry graduated cylinder of 10 mL, and manually tapped with 100 taps for approximately 2 minutes.

The expression force for powders is also an important parameter related to deployment of powders or paste during surgical procedures. The efforts required to expel a liquid from a syringe, and to draw liquid into the syringe, are known as the expression force, and aspiration force respectively. The expression force measure, however, is a more critical for dual-syringe mixing devices. In some embodiments, the composition is capable of being passed through an applicator.

Dual-syringe mixing devices produce a substantially homogenously paste mixture by combining initially separate liquid and solid carriers and then passing the blended contents back and forth between two connected syringes *via* interconnected outlets. Therefore, a low expression force for dispensing the paste from a syringe may be preferred for ease of mixing and ultimately for deployment of the resulting paste. The desired expression force may be less than 1.51 lbf.

As described herein, in some embodiments, the composition may further comprise at least one biologically active agent and non-limiting biologically active agents that may be included in the composition include calcium, as well as therapeutic agents such as antibiotics, anti-inflammatory agents, growth factors, or clotting factors. For example, the composition may further comprise fibrinogen or thrombin.

In some embodiments, the composition may further comprise thrombin.

In some embodiments, the composition further comprises calcium. Calcium is an important element in the clotting cascade. It is needed for activation of factor XIII into factor XIIIa, which cross-links and stabilizes fibrin to generate an insoluble clot.

Calcium used with the invention may be in the form of calcium chloride salt. Alternatively, other salts may be used, such as, calcium acetate and/or calcium citrate. In some embodiments, the calcium source is calcium chloride e.g., in a range of 40-60 mM.

In some embodiments, the composition may comprise more than one biologically active agent, for example, calcium and thrombin.

As used herein, "thrombin" denotes an activated enzyme which results from the proteolytic cleavage of prothrombin (factor II). Thrombin may be produced by a variety of methods of production known in the art, and includes, but is not limited to, recombinant thrombin and plasma derived thrombin.

Human thrombin is a 295 amino acid protein composed of two polypeptide chains joined by a disulfide bond. Both human and non-human (e.g., bovine) thrombin may be used within the scope of the present disclosure.

The composition may further include one or more of the following excipients selected from, without being limited thereto, albumin, saccharides, saccharide derivatives, polyol/s, acetate, citrate, amino acids, polyethylene glycol, and sodium chloride.

The albumin may be in a range of 0.05-1% (w/v) or in a range of 0.5-1% (w/w). The saccharides source may be saccharose and may be e.g., in about 5 g/l concentration.

In some embodiments, the saccharide derivatives source comprises gluconic acid. In some embodiments, the polyol source comprises mannitol e.g., at a concentration of about 2% (w/w). In some embodiments, the acetate source is sodium acetate. In some embodiments, the citrate source can be sodium citrate. In some embodiments, the amino acids comprise histidine. In some embodiments, the polyethylene glycol (PEG) source is PEG-3350 and may be present e.g., at a concentration of about 0.03%, by weight.

"PEG 3350" denotes a PEG compound with an average molecular weight of 3350 Daltons.

Accordingly, in some embodiments, the composition further comprises one or more excipients selected from the group consisting of sodium chloride, mannitol, albumin, and sodium acetate.

In some embodiments, the composition is non-flowable at at-least one temperature around room temperature, such as at one or more temperature values selected from the group consisting of 10°C, 15°C, 20°C, 25°C, 30°C, 35°C, 37°C, and 40°C. This composition is referred to hereinbelow as the "non-flowable" or "not flowable" composition.

As indicated above, the degree of oxidation of the OC may be important to its functional properties such as biocompatibility and bioabsorbability. Products including various degrees of OC oxidation exist, such as a surgical hemostat in which carboxylic acid groups are present at a concentration of 18-21% (by weight) of the oxidized cellulose. On the other hand, OC with a lower concentration of carboxylic acid groups, such as 12%-18% may have superior adhesion prevention properties.

As used herein with reference to OC, the terms "oxidation level", "degree of oxidation", "carboxyl content" and "carboxylation level" are interchangeable, and may be determined per United States Pharmacopeia (USP) 23-NF18.

In some embodiments, the carboxyl content of the OC is 12% to 18% (w/w). In some embodiments, the carboxyl content of the OC is 12-17% (w/w). In some embodiments, the carboxyl content of the OC is 12-16% (w/w).

In some embodiments, the carboxyl content of the OC is about 12%, 13%, 14%, 15%, 16%, 17%, or 18% (w/w) including any value and range therebetween.

The composition, having an OC with a carboxyl content of 12% to 18% is for use as an adhesion prevention composition, for preventing formation of adhesions at a bleeding site of a tissue. In some embodiments, a fabric for preparing the powder is a warp knitted tricot fabric constructed of bright rayon yarn that is subsequently oxidized to include carboxyl or aldehyde moieties in amounts effective to provide the fabrics with biodegradability. As described above, the fabric may be oxidized by reacting the cellulose with a solution of nitrogen dioxide in a perfluorocarbon solvent as described by F. Boardman et al. in US Patent Number 5,180,398.

In another embodiment, a powder of OC/ORC with a carboxyl content (degree of oxidation) ranging from 12% to 18% is for use in a method for adhesion prevention at a bleeding site. In yet another embodiment, the oxidized regenerated cellulose with a carboxyl content (degree of oxidation) ranging from 9.5% to 10.5% is for use in a method for in adhesion prevention at a bleeding site.

In some embodiments, the composition is further treated to obtain a low bioburden, by any method known in the art, such as heat
treatment, radiation treatment, filtration or chemical treatment, e.g., gamma radiation, filtration using a pore size of e.g., 0.22 µm or lower, heat sterilization and aseptic field.

In some embodiments, the method further comprises adding calcium to the composition. In some embodiments, the method further comprises adding at least one biologically active agent to the composition. In some embodiments, the method further comprises adding to the composition one or more excipients selected from sodium chloride, mannitol, albumin, and sodium acetate.

The tissue may be a soft tissue or, e.g., a bone tissue.

The term "soft tissues" as used herein relates to body tissue that is not hardened or calcified. This term especially relates to soft tissues that are vascularized and therefore may be a source of bleeding. Examples for such tissues include but are not limited to connective tissue (such as tendons, ligaments, fascia, skin, fibrous tissues, fat, and synovial membranes), muscles, and internal organs. In general, soft tissues are meant to exclude bone tissue.

The carboxyl content of the OC is at least 9% or above, by weight. The carboxyl content of the OC is equal to or less than 18%, e.g., 18% by weight. The carboxyl content of the OC is 9% to 18%, for example 12% to 18%, by weight.

In some embodiments, the composition is flowable at one or more temperature values selected from the group consisting of 10°C, 15°C, 20°C, 25°C, 30°C, 35°C, 37°C, and 40°C.

In some embodiments, the composition is not flowable at one or more temperature values selected from the group consisting of 10°C, 15°C, 20°C, 25°C, 30°C, 35°C, 37°C, and 40°C.

In some embodiments, the composition is treated to obtain a low bioburden, by a method known in the art, such as, heat treatment, radiation treatment, filtration
or chemical treatment, e.g., gamma radiation, filtration using a pore size of 0.22 µm or lower, heat sterilization and aseptic field.

In some embodiments , the composition further comprises one or more excipients selected from the group consisting of sodium chloride, mannitol, albumin, and sodium acetate.

The composition may be provided in a kit comprising: a) a container containing the composition as described above, b) an applicator for applying the composition to a tissue, and c) optionally instructions for use.

In some embodiments, the container is part of the applicator.

It is appreciated that the consistency of the composition is such that it can be applied, for example, by spreading or by sticking the composition directly onto the bleeding site. Accordingly, the composition does not need to be further spread on or applied to a solid surface, object, or other solid medium such as a strip or a film in order to be in the appropriate form for applying to the bleeding site. Nevertheless, a suitable applicator, such as, for example, a syringe, may be used in order to apply, spread or stick the composition onto the bleeding site, for the purpose of easy access and handling.

The terms "comprises", "comprising", "includes", "including", "having", and their conjugates mean "including but not limited to". The term "consisting of" means "including and limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

In those instances where a convention analogous to "at least one of A, B, and C, etc." is used, in general such a construction is intended in the sense one having skill in the art would understand the convention (e.g., "a composition having at least one of A, B, and C" would include but not be limited to compositions that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Unless otherwise indicated, all numbers such as those expressing, for example, ratios, weight, mole/mole, amounts, viscosity, temperatures, etc., are to be understood as being modified in all instances by the term "about". Accordingly, unless indicated to the contrary, the numerical parameters set forth in this description and attached claims are approximations that may vary by up to plus or minus 10% depending upon the desired properties sought to be obtained by the present invention.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non-limiting fashion.

### Example 1: Adhesion Prevention Efficacy at a Bleeding Site in a Rat Cecal Abrasion Model

Adhesions are a substantial medical problem which can cause chronic pain, infertility and are related to intestinal obstruction that could lead to mortality. Many of these adhesions results from surgical trauma to the peritoneum. To examine the ability of the compositions of to reduce adhesions at a bleeding site, they were tested in an animal model. The chosen modal is a Rat model in which includes cecal abrasion and an abdominal sidewall defect. The model is generally described in Poehnert et al., 2015, International Journal of Medical Sciences 12(1):1-6, with a few minor alterations.

### Materials and Method

The tested groups:
1. *No Treatment (control);*
2. *Group INTERCEED:* commercially available INTERCEED^{®} sheets (Ethicon) was cut to size to be applied to cover the cecum (comparative);
3. *Group INTERCEED*^{®} *powder* is in the form of aggregates and was generated by the process described in US Pat. 10,034,957 Example 1 with the difference that the base material used was INTERCEED^{®} sheets (Ethicon) instead of SURGICEL^{®} sheets (Ethicon). INTERCEED powder in the form of aggregates is also named hereinthroughout: milled/ground INTERCEED in aggregated form, milled/ground INTERCEED which was compressed into small granules, compacted INTERCEED powder, INTERCEED compacted in the form of aggregate.

Briefly, INTERCEED ^{®} had been cut into 1- to 2-inch wide sections before the material was fed into a blade that cuts the fabric into smaller pieces. The cut ORC fabric pieces were then ground into intermediate ORC fine fibers by two consecutive milling processes (hammer milling and air classifier milling). The resulting intermediate ORC fine fibers were then humidified to about 11% to about 16% as measured by Ohaus halogen moisture analyzer and then roller compacted into larger aggregates. The moisture analyzer operated on a thermogravimetric principle wherein the moisture analyzer determined the weight of the sample; the sample was then quickly heated by the integral halogen dryer unit and moisture vaporized. During the drying operation, the instrument continuously determined the weight of the sample and displayed the result.

On completion of drying, a tabulated result was displayed as percent moisture content, percent solids, weight or percent regain, in particular, the analyzer tested between 0.5-1 grams of aggregate with a four-minute ramp, 90°C maximum temperature and the following settings: Test ID - LOD; Profile - Standard; Dry Temperature - 90 °C; Switch Off - A60; Result - Moisture%; Custom - Off; Target Weight - None.

Sieving was done to separate target particles between the size of 75 and 300 microns determined by screen sieving. Excess moisture introduced for purposes of compaction was removed by a dehumidification or drying process after compaction and sieving step for subsequent dosing into applicator devices. Samples were then subjected to the device packaging and sterilization. Storage moisture prior to dosing into an applicator was less than about 2% at conclusion of drying to achieve less than 6% moisture content in controlled environment (0.3-0.6%/hr per 500 g sample moisture gained depending on relative humidity, commonly 25-55% relative humidity) for dosing into applicators.

Taken together, one process for manufacturing the inventive hemostatic aggregates comprised the steps of: a) slitting and cutting of cellulosic source material; b) milling the resulting material from step a); c) a second milling step in an air classifier; d) humidification; e) roller compaction; f) sieving; g) dehumidification or drying; h) optional dosing into storage containers or into delivery devices, primary packaging and secondary packaging; and i) optional sterilization.

The test articles were applied (6 repetitions) to the rat cecal abrasion model as explained herein.

*In vivo Rat cecal abrasion model for testing for adhesion prevention activity:* briefly, animals anesthesia was administered using a single intramuscular injection of a mixture of Ketamine HCl 80 mg/kg (Fort Dodge Pty. Ltd., Australia) and Xylazine HCl 10 mg/kg (VMD, Belgium; the anesthesia was administered intramuscularly and not intraperitoneally because the operating procedure was performed on the peritoneal cavity).

A 6 cm incision mark was made on the skin overlaying the linea on the ventral midline. The ventral skin was shaved, prepared with iodophor solution and incised. The skin was retracted and undermined slightly to facilitate suturing at the end of the procedure. With the muscle wall exposed, a 5 cm incision in the muscle was made along the linea all through the peritoneal cavity. The right abdominal wall was reflected. A 1x2 cm layer of the peritoneum and part of the muscle was removed. The medial edge of this defect was located 1 cm lateral from the midline incision and parallel to it. The abdominal wall defect was monitored to observe bleeding. A corresponding defect was made on the cecum, by scraping with a scalpel so that a homogeneous surface of petechial hemorrhages was formed over a 1x2 cm area. Prior to abrasion, the cecum was elevated and positioned so that upon closure, the cecum would contact the abdominal wall defect to induce local adhesion. The two surfaces were air-dried for 10 minutes. For each test article, an amount was spread over the cecum to fully cover the cecum with a thin layer of the test article. The "no treatment" group was left untouched. After the group assignment and application, the cecum and abdominal wall defect were held together for 1 minute prior to closure. Organs were replaced anatomically, and two sutures were placed in each end of the defects to maintain organs proximity. During the treatment, the animals were observed carefully to remove any animal with unexpected response to the anesthetic treatment.

Following the procedure, the animals were given a single dose of butorphanol tartrate (Torbugestic) 0.5-2.0 mg/kg body weight to reduce pain. The animals were monitored daily, and in cases where clinical signs or behavior changes were observed, butorphanol 0.5-2.0 mg/kg body weight was administered to the animals subcutaneously every four hours as necessary to control the pain, depending on activity of the animal. Fourteen days following surgery, animals were euthanized by CO₂ asphyxiation. The abdomen was opened, and the surgical site inspected. Adhesions were graded by a blinded observer.

The adhesions strength to the various abdominal organs were evaluated according to the following scheme (Poehnert et al., 2015, International Journal of Medical Sciences 12(1): 1-6): Grade 0 - no adhesion; Grade 1- filmy adhesions easily removed; Grade 2 - can be removed with blunt dissection; Grade 3 - requires sharp dissection; Grade 4 - highly inseparable with difficulty to differentiate edge of different tissues. In addition to the adhesion strength, adhesion coverage was measured using a ruler. Adhesion intensity was calculated as adhesion strength X adhesion coverage (mm).

As can be seen in **Fig. 1****,** INTERCEED powder ORC (Group 3) exhibited adhesion prevention potency of about 135% as compared to ORC fabric (Group 2).

## Claims

1. A composition comprising oxidized cellulose (OC) in the form of a powder, for use as an adhesion prevention material in the presence of blood, wherein the carboxyl content of the OC is 9% to 18% by weight.

2. The composition for use of claim 1, wherein the OC comprises oxidized regenerated cellulose (ORC).

3. The composition for use of any one of claims 1 to 2, wherein:
(i) the powder is in an aggregated form; and/or
(ii) the powder comprises milled OC.

4. The composition for use of any one of claims 1 to 3, wherein the powder is gamma irradiated.

5. The composition for use of any one of claims 1 to 4, wherein the OC comprises ORC in the form of a compacted powder, optionally wherein the ORC powder comprises a roller compaction processed ORC powder, and/or a hammer mill processed ORC powder.

6. The composition for use of any one of claims 1 to 5, further comprising an additive, wherein said additive comprises one or more members selected from CMC or other polysaccharides, calcium salt, anti-infective agent, hemostasis promoting agent, gelatin, collagen.

7. The composition for use of any one of claims 1 to 6, wherein the powder has a bulk density greater than 0.45 g/mL.

8. The composition for use of any one of claims 1 to 7, wherein the powder has sphericity (sh50) equal to or greater than 0.7.

9. The composition for use of claim 8, wherein the OC forms interconnected individual cellulosic fibrils having, in the aggregate form, a sphericity of at least 0.6, and a dimension along its longest axis that is less than 500 microns and greater than 50 microns.

10. The composition of any one of claims 1 to 9, wherein the carboxyl content of the OC is at least 12%.

11. The composition for use of any one of claims 1 to 10, wherein the composition is provided in a kit comprising:
a. a container containing the composition for use as defined in any one of claims 1 to 10;
b. an applicator for applying the composition to a tissue; and
c. optionally instructions for use.

12. The composition for use of claim 11, wherein the container is comprised in the applicator.

## Patentansprüche

1. Zusammensetzung, umfassend oxidierte Cellulose (OC) in Form eines Pulvers, zur Verwendung als ein Adhäsionspräventionsmaterial in Gegenwart von Blut, wobei der Carboxylgehalt der OC 9 Gew.-% bis 18 Gew.-% beträgt.

2. Zusammensetzung zur Verwendung nach Anspruch 1, wobei die OC oxidierte regenerierte Cellulose (ORC) umfasst.

3. Zusammensetzung zur Verwendung nach einem der Ansprüche 1 bis 2, wobei:
(i) das Pulver in einer aggregierten Form vorliegt; und/oder
(ii) das Pulver gemahlene OC umfasst.

4. Zusammensetzung zur Verwendung nach einem der Ansprüche 1 bis 3, wobei das Pulver gammabestrahlt ist.

5. Zusammensetzung zur Verwendung nach einem der Ansprüche 1 bis 4, wobei die OC ORC in Form eines verdichteten Pulvers umfasst, gegebenenfalls wobei das ORC-Pulver ein mittels Walzverdichtung verarbeitetes ORC-Pulver und/oder ein mittels Hammermühle verarbeitetes ORC-Pulver umfasst.

6. Zusammensetzung zur Verwendung nach einem der Ansprüche 1 bis 5, ferner umfassend ein Additiv, wobei das Additiv ein oder mehrere Elemente umfasst, die aus CMC oder anderen Polysacchariden, Calciumsalz, einem Antiinfektivum, einem hämostasefördernden Mittel, Gelatine, Kollagen ausgewählt sind.

7. Zusammensetzung zur Verwendung nach einem der Ansprüche 1 bis 6, wobei das Pulver eine Schüttdichte, die größer als 0,45 g/mL ist, aufweist.

8. Zusammensetzung zur Verwendung nach einem der Ansprüche 1 bis 7, wobei das Pulver eine Sphärizität (sh50), die gleich oder größer als 0,7 ist, aufweist.

9. Zusammensetzung zur Verwendung nach Anspruch 8, wobei die OC miteinander verbundene einzelne cellulosische Fibrillen ausbildet, die in der Aggregatform eine Sphärizität von mindestens 0,6 und eine Abmessung entlang ihrer längsten Achse aufweisen, die kleiner als 500 Mikrometer und größer als 50 Mikrometer ist.

10. Zusammensetzung nach einem der Ansprüche 1 bis 9, wobei der Carboxylgehalt der OC mindestens 12 % beträgt.

11. Zusammensetzung zur Verwendung nach einem der Ansprüche 1 bis 10, wobei die Zusammensetzung in einem Kit bereitgestellt ist, umfassend:
a. einen Behälter, der die Zusammensetzung zur Verwendung nach einem der Ansprüche 1 bis 10 beinhaltet;
b. einen Applikator zum Auftragen der Zusammensetzung auf ein Gewebe; und
c. gegebenenfalls Anweisungen zur Verwendung.

12. Zusammensetzung zur Verwendung nach Anspruch 11, wobei der Behälter in dem Applikator umfasst ist.

## Revendications

1. Composition comprenant de la cellulose oxydée (OC) sous la forme d'une poudre, pour utilisation en tant que matériau de prévention des adhérences en présence de sang, dans laquelle la teneur en carboxyle de l'OC est de 9 % à 18 % en poids.

2. Composition pour utilisation selon la revendication 1, dans laquelle l'OC comprend de la cellulose régénérée oxydée (ORC).

3. Composition pour utilisation selon l'une quelconque des revendications 1 à 2, dans laquelle :
(i) la poudre est sous une forme agrégée ; et/ou
(ii) la poudre comprend de l'OC broyé.

4. Composition pour utilisation selon l'une quelconque des revendications 1 à 3, dans lequel la poudre est irradiée aux rayons gamma.

5. Composition pour utilisation selon l'une quelconque des revendications 1 à 4, dans laquelle l'OC comprend de l'ORC sous la forme d'une poudre compactée, éventuellement dans laquelle la poudre d'ORC comprend une poudre d'ORC traitée par compactage à rouleaux et/ou une poudre d'ORC traitée par un broyeur à marteaux.

6. Composition pour utilisation selon l'une quelconque des revendications 1 à 5, comprenant en outre un additif, dans laquelle ledit additif comprend un ou plusieurs éléments choisis parmi la CMC ou d'autres polysaccharides, un sel de calcium, un agent anti-infectieux, un agent favorisant l'hémostase, la gélatine, le collagène.

7. Composition pour utilisation selon l'une quelconque des revendications 1 à 6, dans laquelle la poudre a une masse volumique apparente supérieure à 0,45 g/mL.

8. Composition pour utilisation selon l'une quelconque des revendications 1 à 7, dans laquelle la poudre présente une sphéricité (sh50) égale ou supérieure à 0,7.

9. Composition pour utilisation selon la revendication 8, dans laquelle l'OC forme des fibrilles cellulosiques individuelles interconnectées ayant, sous forme d'agrégat, une sphéricité d'au moins 0,6, et une dimension le long de son axe le plus long qui est inférieure à 500 microns et supérieure à 50 microns.

10. Composition selon l'une quelconque des revendications 1 à 9, dans laquelle la teneur en carboxyle de l'OC est d'au moins 12 %.

11. Composition pour utilisation selon l'une quelconque des revendications 1 à 10, dans laquelle la composition est fournie dans un kit comprenant :
a. un récipient contenant la composition pour utilisation selon l'une quelconque des revendications 1 à 10 ;
b. un applicateur pour l'application de la composition sur un tissu ; et
c. éventuellement, des instructions pour l'utilisation.

12. Composition pour utilisation selon la revendication 11, dans laquelle le récipient est compris dans l'applicateur.
